**Europäisches Patentamt**

(19) **European Patent Office**

**Office européen des brevets**

(11) Veröffentlichungsnummer: **0 129 058**

**B1**

(12) **EUROPÄISCHE PATENTSCHRIFT**

(45) Veröffentlichungstag der Patentschrift:
23.07.86

(51) Int. Cl.⁴: **C 07 C 51/10,** C 07 C 53/08,
C 07 C 53/122, C 07 C 53/124

(21) Anmeldenummer: 84105507.2

(22) Anmeldetag: 15.05.84

(54) Verfahren zur Herstellung von Gemischen aus Essigsäure, Propionsäure und Buttersäure.

(30) Priorität: 19.05.83 DE 3318210

(43) Veröffentlichungstag der Anmeldung:
27.12.84 Patentblatt 84/52

(45) Bekanntmachung des Hinweises auf die Patenterteilung:
23.07.86 Patentblatt 86/30

(84) Benannte Vertragsstaaten:
BE DE FR GB IT NL

(56) Entgegenhaltungen:
EP-A-0 021 241
EP-A-0 030 110
EP-A-0 065 398
US-A-3 407 220

(73) Patentinhaber: HOECHST AKTIENGESELLSCHAFT,
Postfach 80 03 20, D-6230 Frankfurt am Main 80
(DE)

(72) Erfinder: Leupold, Ernst Ingo, Dr., Am Zäunefeld
15, D-6392 Neu-Anspach (DE)
Erfinder: Schmidt, Hans-Joachim, Dr., Am
Burgenblick 6, D-6240 Königstein-Taunus (DE)

EP 0 129 058 B1

LIBER, STOCKHOLM 1986

## Beschreibung

Die vorliegende Erfindung betrifft ein Verfahren zur Herstellung von Gemischen aus Essigsäure, propionsäure und Buttersäure.

Bei der CO-Hydrierung an heterogenen Rhodium-Katalysatoren gemäß den deutschen Offenlegungsschriften 2 814 365, 2 712 732, 2 846 148, 2 924 962 und 3 038 448 erhalt man Gemische sauerstoffhaltiger Verbindungen in Form einer wäßrigen Phase.

Weiterhin wurde bereits in der deutschen Patentanmeldung P 3 203 060.6 beschrieben, daß mit Rhodiumkatalysatoren, die Verbindungen der Seltenen Erden und der Alkalimetalle enthalten, in außerordentlich hoher Selektivität sauerstoffhaltige Verbindungen erhalten werden können. Beispielsweise zeichnen sich mit Ytterbium- und Lithiumverbindungen dotierte Rhodiumkatalysatoren durch hohe Selektivität bei nahezu unbegrenzter Lebensdauer aus. Es fällt ein Kondensat mit hohem Gehalt (bis über 70 Gew.-%) an sauerstoffhaltigen Verbindungen an. Dieses enthält einen überraschend hohen Anteil an Essigsäure und außerdem hauptsächlich Acetaldehyd, Ethanol und Ethylacetat. Daneben sind in einer Größenordnung von insgesamt bis zu 5 Gew.-% weitere sauerstoffhaltige Verbindungen mit drei oder mehr Kohlenstoffatomen enthalten, wie propionaldehyd, Butyraldehyd, n-Propanol, n-Butanol, Essigsäurepropylester, Essigsäurebutylester.

Die vollständige destillative Trennung des Gemischs ist wegen der vielfachen Azeotropbildung kaum durchführbar. Andererseits sind auch nicht alle der genannten produkte von großem technischen Interesse, Außerdem stößt die sinnvolle Verwendung einer Vielzahl von Koppelprodukten bekanntermaßen auf Schwierigkeiten.

Somit bestand die Aufgabe, das produktspektrum so zu vereinfachen, daß nur noch erwünschte, technisch interessante produkte übrig bleiben.

Es wurde nun gefunden, daß man das Produktspektrum durch eine Oxidation in flüssiger Phase an einem heterogenen Platinkatalysator wesentlich vereinfachen kann, so daß nur noch die für die chemische Industrie sehr wichtigen Zwischenprodukte Essigsäure, Propionsäure und Buttersäure erhalten weiden.

Gegenstand der Erfindung ist daher ein Verfahren zur Herstellung von Gemischen aus Essigsäure, Propionsäure und Buttersäure, dadurch gekennzeichnet, daß man Synthesegas in Gegenwart eines Rhodiumkatalysators, der als Promotor mindestens eine Verbindung von Mg, $M_n$, La, einem Seltenen Erdmetall, Sc, Y, W, Fe, Zr, Cr, Hf, Pt, U sowie als Aktivater mindestens eine Verbindung eines Alkalimetalls enthält, umsetzt und anschließend das Umsetzungsprodukt kondensiert und die wäßrige Phase des Kondensats, gegebenenfalls nach Abtrennung des Acetaldehyds, mit Sauerstoff oder einem sauerstoffhaltigen Gas in Gegenwart eines Platinkatalysators bei einem pH $\leq$ 7 oxidiert.

Im ersten Schritt des erfindungsgemäßen Verfahrens werden Kohlenmonoxid und Wasserstoff in hoher Selektivität zu Essigsäure, Acetaldehyd und Ethanol umgesetzt. Daneben entstehen in geringen Mengen auch solche produkte, die durch eine Folgereaktion, zum Beispiel durch Veresterung, Acetalisierung oder Kondensation gebildet werden. Hierzu zählen vor allem Ethylacetat und das Diethylacetal des Acetaldehyds. Der Anteil an anderen sauerstoffhaltigen Vorbindungen mit drei oder mehr Kohlenstoffatomen im Molekül ist sehr gering und liegt normalerweise unter 5 Mol-%, bezogen auf umgesetztes Kohlenmonoxid. Die Gesamtselektivität zu sauerstoffhaltigen $C_2$-Verbindungen, einschließlich der in Ethylacetat und Acetylaldehyddiethylacetal umgewandelten Produkte, beträgt bis zu 82 %, bezogen auf umgesetztes Kohlenmonoxid. Der Rest des umgesetzten Kohlenmonoxids wird im wesentlichen in Methan und andere gasförmige Kohlenwasserstoffe und im geringen Maße in Kohlendioxid umgewandelt.

Zum Aufbau des Katalysators für die Synthesegas-Umsetzung kann man von Salzen oder Komplexverbindungen des Rhodiums ausgehen. Rhodium kann auf dem Träger in metallischer Form oder auch in einer Wertigkeitsstufe unter drei, also als Komplexverbindung des nulkwertigen Rhodiums oder als Salz oder Komplexverbindung des ein- oder zweiweitigen Rhodiums vorliegen. Geeignet sind z.B. Chloride, Bromide und Jodide des Rhodiums oder auch Doppelsalzw des Rhodiums mit Alkalihalogeniden, wie z.B. Dikaliumtiichlororhodat. Geeignet sind ferner Komplexverbindungen, die neben Rhodium und Halogen noch komplexbildende Liganden, wie Trialkylphosphine, Triarylphosphine, Kohlenmonoxid, Olefine oder Wasser enthalten, also z.B. Tris-triphenylphosphin-rhodium-I-chlorid, -bromid oder -jodid, Tristriphenylphosphinrhodium-III-chlorid, Bis-tri-o-tolyl-phosphin-rhodium-II-chlorid, Carbonyl-bis-triphenyl-phosphin-rhodium-I-bromid oder Dicäsiumcarbonyl-pentachlororhodat-III. Darüberhinaus kommen auch solche Verbindungen des Rhodiums in Betracht, in denen es ionogen oder komplex an einen Träger gebunden ist. Beispiele hierfür sind die mit Rhodiumhalogeniden ausgetauschten Zeolithe und Ionenaustauscher.

Die Katalysatoren enthalten neben Rhodium noch Promotoren und Aktivatoren. Als Promotoren werden Verbindungen der folgenden Elemente eingesetzt: Magnesium, Mangan, Lanthan, eltene Erdmetalle (Ordnungszahl 58-71), Scandium, Yttrium, olfram, Eisen, Zirkon, Chrom, Hafnium, Platin, Uran. Geeignete Verbindungen sind beispielsweise die Chloride, Bromide, Fluoride, Nitrate, Acetate, Oxalate, Acetylacetonate oder Taitrate. Bevorzugt verwendet man die Chloride, Bromide, Acetate und Acetylacetonate dieser Elemente, vor allem die Chloride oder Bromide. Ein besonders geeigneter Promotor ist Ytterbium in Form der genannten Verbindungen.

Als Aktivatoren werden die Oxide, Salze oder Komplexverbindungen des Lithiums, Natriums, Kaliums, Rubidiums oder Cäsiums oder deren Gemische verwendet, also beispielsweise die Oxide, Hydroxide, Carbonate, Chloride, Bromide, Jodide, Nitrate, Acetate, Silikate und/oder Aluminate der Alkalimetalle. Bevorzugt sind Natriumverbindungen und Lithiumverbindungen, vor allem letztere.

Als Katalysatorträger werden übliche Trägermaterialien mit unterschiedlichen spezifischen Oberflächen verwendet. Allerdings werden Träger mit spezifischen Oberflächen von 50 bis 1000 m /g bevorzugt. Geeignet sind z.B. Kieselsäure, natürliche oder synthetische Silikate von Elementen der II. bis VIII. Gruppe des Periodischen Systems (also beispielsweise die Silikate des Magnesiums, Calciums, Aluminiums, Mangans), ferner Aluminiumoxid, Thoriumdioxid, Zeolithe und Spinelle. Vorzugsweise verwendet man Kieselsäure oder Silikate.

Zur Herstellung der Katalysatoren werden die Rhodiumverbindung, der Promotor und der Aktivator gleichzeitig oder in aufeinanderfolgenden Stufen in beliebiger Reihenfolge auf den Träger aufgebracht, und zwar im allgemeinen durch Tränkung des Trägers mit Lösungen der aktiven Komponenten in geeigneten Lösungsmitteln, wie z.B. Wasser, Alkohol, Aceton, Acetylaceton oder Essigsäure und nachfolgendes Trocknen.

Das Alkalimetall und die Promotoren können aber auch in eine Gerüstsubstanz eingebaut sein, beispielsweise in eine Silikat oder Aluminiumoxid enthaltende Trägersubstanz, wie Kieselsäure, Aluminiumoxid oder Aluminiumsilikat. Eine weitere vorteilhafte Möglichkeit besteht darin, das Alkalimetall oder die Promotoren mittels Ionenaustausch an Kationenaustauscher zu binden, die auch als Träger für das Rhodium geeignet und unter den Versuchsbedingungen beständig sind, beispielsweise die als Molsiebe bekannten natürlichen oder synthetischen Aluminiumsilikate.

Vorzugsweise wird der Katalysator vor Beginn der Synthesegas-Umsetzung noch reduziert. Als Reduktionsmittel kommen beispielsweise Wasserstoff, Kohlenmonoxid, Methanol oder Aceton in Betracht. Diese Reduktion kann in einer getrennten Apparatur oder im Reaktor selbst durchgeführt werden. Vorzugsweise wird bei Temperaturen unter 300°C, insbesondere zwischen 100 und 275°C, reduziert. Vielfach ist es zweckmäßig, die Reduktion nicht mit den unverdünnten reduzierend wirkenden Gasen, sondern mit einem zusätzlichen Anteil an Inertgasen, wie z.B. Stickstoff, Kohlendioxid oder auch Edelgasen, vorzunehmen.

Die Konzentration an Rhodium, Promotoren und Aktivatoren in den Katalysatoren kann in weiten Grenzen variiert werden; im Allgemeinen liegen die Werte, bezogen auf die Metalle, zwischen 0,1 und 15 Gew.-% für Rhodium, zwischen 0,1 und 20 Gew.-% für die Promotoren und zwischen 0,01 und 5 Gew.-% für die Alkalimetalle. Bevorzugt sind Katalysatoren mit 1,0 bis 10 Gew-% Rhodium und 0,1 bis 10 Gew-% Promotor(en).

Zur Durchführung der Synthesegas-Umsetzung werden Gasgemische, die ganz oder zu einem überwiegenden Teil aus Kohlenmonoxid und Wasserstoff bestehen und daneben gegebenenfalls noch andere Komponenten wie Stickstoff, Argon, Kohlendioxid oder Methan enthalten können, über den Katalysator geleitet. Das molare Verhältnis von Kohlenmonoxid zu Wasserstoff kann dabei in weiten Grenzen variiert werden. Bevorzugt sind Molverhältnisse zwischen 5:1 und 1:5 und besonders zwischen 3:1 und 1:3.

Die Reaktionstemperaturen liegen im Allgemeinen zwischen 175 und 400°C, vorzugsweise zwischen 200 und 380°C, und die Reaktionsdrücke zwischen 1 und 300 bar, vorzugsweise zwischen 10 und 200 bar.

Zweckmäßig ist es, Temperatur und Druck so aufeinander abzustimmen, daß eine hohe Selektivität zu den gewünschten Verbindungen gewährleistet ist und die bei höheren Temperaturen begünstigte exotherme Bildung von Methan geringgehalten wird. Man wird deshalb hohe Drücke und möglichst niedrige Temperaturen bevorzugen.

Für die Synthesegas-Umsetzung ist die Gasphase bevorzugt. Hierzu können die herkömmlichen Festbettreaktoren verwendet werden, wobei es zur besseren Wärmeabführung vorteilhaft ist, die Katalysatorschichtdicke gering zu halten. Ferner sind auch Reaktoren mit bewegtem Katalysatorbett oder Wirbelbettreaktoren geeignet.

Eine besonders bevorzugte Ausführungsform der Synthesegas-Umsetzung besteht darin, sie in einer Kreisgasapparatur in der Gasphäse durchzuführen, in der nach Abtrennung der kondensierbaren Reaktionsprodukte das nicht umgesetzte Gasgemisch wieder in den Reaktor zurückgeführt wird.

Diese Verfahrensweise ist besonders wirtschaftlich und ermöglicht durch Verdünnung des Frischgases mit dem im Kreislauf zurückgeführten wasserstoffärmeren Restgas höhere Reaktionstemperaturen und damit höhere Raum-Zeit-Ausbeuten bei unveränderten Selektivitäten. Als Kreisgasapparaturen können dabei solche mit innerem oder äußerem Gasumlauf in Betracht kommen.

Anschließend an die Synthesegas-Umsetzung wird das Umsetzungsprodukt kondensiert und die wäßrige Phase des Kondensats, gegebenenfalls nach Abtrennung des Acetaldehyds, mit Sauerstoff odei einem sauerstoffhaltigen Gas in Gegenwart eines Platinkatalysators bei einem pH ⩽ 7 oxidiert.

Der Befund, daß sich die in der wäßrigen Phase des Kondensats der Rh-katalysierten CO-hydrierung enthaltenen Aldehyde, Alkohole und Ester in einer Stufe mit hohem Umsetzungsgrad zu einem Gemisch aus Essigsäure, Propionsäure und Buttersäure umwandeln lassen, war überraschend. Zwar ist die homogenkatalysierte Oxidation eines Aldehyds und die heterogenkatalysierte Oxidation eines Alkohols bekannt und technisch erprobt. Jedoch wurde bisher die einstufige Oxidation eines Gemisches mit jeweils mehreren Aldehyden, Alkoholen und Estern in Gegenwart von Carbonsäuren noch nicht beschrieben. Da die Umsetzung bei pH $\leqslant$ 7 durchgeführt wird, entfällt der Zusatz von Basen in molaren Mengen. Somit ist eine zu unerwünschter Salzbildung führende Neutralisation ebenso überflüssig wie die Extraktion des Produktes mit einem Extraktionsmittel, so daß das erfindungsgemäße Verfahren sehr wirtschaftlich ist. Während der Oxidation verringert sich der pH-Wert kontinuierlich von 7 auf einen kleineren Wert, welcher von der am Ende erzielten Konzentration der Carbonsäuren abhängt.

Bevorzugtes Oxidationsmittel ist reiner Sauerstoff; es können jedoch auch Mischungen von Sauerstoff mit Inertgasen, wie Luft, verwendet werden.

Als Platinkatalysatoren eignen sich handelsübliche Trägerkatalysatoren, insbesondere Aktivkohlen mit 5 bis 10 Gew.-% Platin.

Die Anwendung von Druck ist nicht zwingend erforderlich, jedoch steigt die Reaktionsgeschwindigkeit mit dem Sauerstoffpartialdruck deutlich an. Bevorzugt ist daher ein Druckbereich von 1 bis 10 bar (absolut); auch bei höherem Druck, z.B. 100 bar, läuft die Oxidation zufriedenstellung ab Allerdings kann dann der Vorteil der höheren Reaktionsgeschwindigkeit duich höhere Investitionen aufgehoben werden.

Im Allgemeinen enthält die eingesetzte wäßrige Phase des Kondensates aus der CO-Hydrierung 20 - 50 Gew.-% Wasser und 50 - 80 Gew.-% sauerstoffhaltige organische Verbindungen.

Vorzugsweise verdünnt man das Kondensat vor der Oxidation auf einen Wassergehalt von 70 - 90 Gew.-%. Ein geringerer Wassergehalt ist prinzipiell möglich, jedoch nimmt die Reaktionsgeschwindigkeit mit steigendem Gehalt an organischen Verbindungen ab. Ein höherer Wassergehalt ist ebenfalls möglich; allerdings steigt damit der Aufwand für die Isolierung der entstehenden Carbonsäuren.

Die Oxidationstemperatur kann in weiten Grenzen schwanken, jedoch ist ein Bereich von 30 bis 75°C, insbesondere von 50 bis 70°C bevorzugt, weil dann eine besonders hohe Selektivität und Reaktivität erzielt wird.

Das Verfahren ist insofern flexibel, als - wenn gewünscht - sowohl Acetaldehyd (als niedigstsiedende Komponente) wie auch Essigsäure zusammen mit bereits bei der CO-Hydrierung in Spuren entstandener Propionsäure und Buttersäure (als höchstsiedende Fraktion) vor der Oxidation ganz oder teilweise destillativ abgetrennt werden können. Die Abtrennung des Acetaldehyds ist beispielsweise von Interesse, wenn dieser als Zwischenprodukt z.B. für Kondensationsreaktionen eingesetzt werden kann. Die Abtrennung der Essigsäure verringert das Volumen des Oxidationsreaktors, andererseits ist jedoch immer ein gewisser Anteil zur Aufrechterhaltung einer homogenen Lösung notwendig, so daß höchstens eine teilweise Abtrennung der Essigsäure in Frage kommt.

Die erfindungsgemäße Oxidation kann in allen Apparaturen, die sich für die Durchführung von Reaktionen in der Flüssigphase mit oder ohne Anwendung von Überdruck eignen, durchgeführt werden, beispielsweise in einem Rührkessel oder in einer Blasensäule mit suspendiertem Katalysator; es können aber auch Festbettreaktoren mit gekörntem Katalysator als Rieselphasenreaktoren eingesetzt werden. Das Reaktionsgemisch kann nach bekannten Methoden aufgearbeitet werden. Zweckmäßig ist eine fraktionierte Destillation, wobei das Wasser zuerst abgetrennt wird (das noch geringe Mengen an unumgesetzten Aldehyden, Alkoholen und Estern enthalten kann).

**Beispiel 1**

Ein durch Imprägnierung eines Kieselsäureträgers mit den Chloriden des Rhodiums, des Magnesiums und des Natriums hergestellter Katalysator enthält nach Trocknung und Reduktion mit Wasserstoff bei 275°C 3.0 Gew.-% Rh, 0.4 Gew.-% Na und 0.2 Gew.-% Mg. Über diesen Katalysator wird in einem Strömungsrohr bei 20 bar und einer Temperatur von 275°C kontinuierlich Synthesegas (40 Vol.% CO, 59 Vol.% $H_2$, 1 Vol.% $CO_2$) bei einer Raumgeschwindigkeit von 700 h geleitet. Das Reaktionsprodukt wird kondensiert. Die wäßrige Phase des Kondensats enthält 39 Gew.-% Wasser; durch Verdünnen wird ihr Wassergehalt auf 75 Gew.-% erhöht. Die Zusammensetzung ist dann wie in der Tabelle angegeben.

300 g der verdünnten wäßrigen Phase werden zusammen mit 15 g eines handelsüblichen Katalysators (5 % Platin auf Aktivkohle) in ein von außen beheiztes senkrecht angeordnetes Glasrohr (Durchmesser 20 mm, Länge 500 mm) gefüllt. Durch eine Glasfritte leitet man 10 Stunden lang von unten 10 Nl/h Sauerstoff bei einer Temperatur von 50°C ein. Die filtrierte Reaktionslösung enthält die in der Tabelle angegebenen Mengen Essigsäure, Propionsäure und Buttersäure.

**Tabelle**

|  | Zusammensetzung (Gew. —%) | |
|---|---|---|
|  | vor der Oxidation | nach der Oxidation |
| Acetaldehyd | 2.8 | < 0.1 |
| Propionaldehyd | 0.8 | < 0.1 |
| Butyraldehyd | 0.3 | < 0.1 |
| Essigsäureethylester | 1.9 | < 0.1 |
| Ethanol | 2.1 | < 0.1 |
| Essigsäurepropylester | 0.9 | < 0.1 |
| Propanol | 0.5 | < 0.1 |
| Essigsäurebutylester | 0.4 | < 0.1 |
| n-Butanol | 0.2 | < 0.1 |
| Essigsäure | 13.5 | 21.5 |
| Propionsäure | 0.4 | 1.6 |
| Buttersäure | < 0.1 | 0.1 |
| Wasser | Rest | Rest |

**Beispiel 2**

Ein analog Beispiel 1 hergestellter Synthesegas-Katalysator enthält 2.9 Gew.-% Rh, 0.3 Gew.-% Ytterbium und 0.05 Gew.-% Li. Dieser Katalysator wird wie im Beispiel 1, jedoch bei 300°C eingesetzt. Die wäßrige Phase des Kondensats wird einer kontinuierlichen Destillation bei Normaldruck unterworfen, wobei die Hauptmenge an Acetaldehyd bei 20°C über Kopf geht. Das Sumpfprodukt der Destillation wird durch Verdünnung auf einen Wassergehalt von 75 Gew.-% eingestellt (Zusammensetzung s. Tabelle).

300 g des Sumpfprodukts werden wie in Beispiel 1 der katalytischen Oxidation unterworfen. Bei 65°C werden 6 Stunden lang 5 Nl/h Sauerstoff eingeleitet. Die filtrierte Lösung enthält die in der Tabelle angegebenen Mengen der Carbonsäuren.

**Tabelle**

|  | Zusammensetzung (Gew. —%) | |
|---|---|---|
|  | vor der Oxidation | nach der Oxidation |
| Acetaldehyd | 0.3 | < 0.1 |
| Propionaldehyd | 0.4 | < 0.1 |
| Butyraldehyd | 0.2 | < 0.1 |
| Essigsäureethylester | 3.1 | < 0.1 |
| Ethanol | 1.7 | < 0.1 |
| Essigsäurepropylester | 0.3 | < 0.1 |
| Propanol | < 0.1 | < 0.1 |
| Essigsäurebutylester | 0.4 | < 0.1 |
| n-Butanol | < 0.1 | < 0.1 |
| Essigsäure | 16.5 | 20.1 |
| Propionsäure | 0.1 | 0.6 |
| Buttersäure | < 0.1 | 0.5 |
| Wasser | Rest | Rest |

**Patentansprüche**

1. Verfahren zur Herstellung von Gemischen aus Essigsäure, Propionsäure und Buttersäure, dadurch gekennzeichnet, daß man Synthesegas in Gegenwart eines Rhodiumkatalysators, der als Promotor mindestens eine Verbindung von Mg, Mn, La, einem Seltenen Erdmetall, Sc, Y, W, Fe Zr, Cr, Hf, Pt, U sowie als Aktivator mindestens eine Verbindung eines Alkalimetalls enthält, umsetzt und anschließend das Umsetzungsprodukt kondensiert und die wäßrige Phase des Kondensats, gegebenenfalls nach Abtrennung des Acetaldehyds, mit Sauerstoff oder einem sauerstoffhaltigen Gas in Gegenwart eines Platinkatalysators bei einem pH < 7 oxidiert.

2. Verfahren nach Anspruch 1, dadurch gekennzeichnet, daß man den Wassergehalt der in die Oxidationsreaktion eingesetzten wäßrigen Phase auf 70 bis 90 Gew.-% einstellt.

3. Verfahren nach Anspruch 1 oder 2, dadurch gekennzeichnet, daß man die Oxidation bei einem absoluten Druck von 1 bis 20 bar durchführt.

4. Verfahren nach einem der Ansprüche 1 bis 3, dadurch gekennzeichnet, daß man die Oxidation bei 30 bis 75°C durchführt.

**Claims**

1. A process for the preparation of mixtures of acetic acid, propionoc acid and butyric acid, which comprises reacting synthesis gas in the presence of a rhodium catalyst containing, as a promoter, at least one compound of Mg, Mn, La, a rare-earth metal, Sc, Y, W, Fe, Zr, Cr, Hf, Pt and U and also, as an activator, at least one compound of an alkali metal, and subsequently condensing the reaction product and oxidizing the aqueous phase of the condensate, if appropriate after removing the acetaldehyde, with oxygen or an oxygen-containing gas in the presence of a platinum catalyst at a pH, < 7.

2. The process as claimed in claim 7, wherein the water content of the aqueous phase employed in the oxidation reaction is adjusted to 70 to 90% by weight.

3. The process as claimed in claim 7 or 2, wherein the oxidation is carried out under an absolute pressure of 7 to 20 bar.

4. The process as claimed in any one of claims 7 to 3, wherein the oxidation is carried out at 30 to 75°C.

**Revendications**

1.- Procédé de préparation de mélanges d'acide acétique, d'acide propionique et d'acide butyrique, procédé caractérisé en ce qu'on fait réagir un gaz de synthèse en présence d'un catalyseur au rhodium qui contient, comme promoteur, au moins un composé d'un élément pris dans l'ensemble constitué par Mg, Mn, La, les métaux des terres rares, Sc, Y, W, Fe, Zr, Cr, Hf, Pt et U et, comme activant, au moins un composé d'un métal alcalin, puis on condense le produit de réaction et on oxyde la phase aqueuse du condensat, éventuellement après en avoir separé l'acétaldéhyde, par de l'oxygène ou par un gaz contenant de l'oxygène, en présence d'un catalyseur au platine, à un pH inférieur ou égal à 7.

2.- Procédé selon la revendication caractérisé en ce que la teneur en eau de la phase aqueuse mise en jeu dans la réaction d'oxydation est ajustée entre 70 et 90 % en poids.

3,- Procédé selon l'une des revendications 1 et 2, caractérisé en ce que l'oxydation est effectuée sous une pression absolue de 1 à 20 bar.

4.- Procédé selon l'une quelconque des revendications 1 à 3, caractérisé en ce que l'oxydation est effectuée à une température de 30 à 75°C.